(19) Europäisches Patentamt
European Patent Office
Office européen des brevets

(11) Publication number: **0 280 031 B1**

(12) # EUROPEAN PATENT SPECIFICATION

(45) Date of publication of patent specification: **23.03.94**

(51) Int. Cl.5: **G03C 1/10**, //C07C335/00

(21) Application number: **88100710.8**

(22) Date of filing: **20.01.88**

(54) **Silver halide photosensitive material containing thiourea or analogue compound.**

(30) Priority: **25.02.87 US 18388**

(43) Date of publication of application:
**31.08.88 Bulletin 88/35**

(45) Publication of the grant of the patent:
**23.03.94 Bulletin 94/12**

(84) Designated Contracting States:
**BE DE FR GB**

(56) References cited:
**EP-A- 0 115 351**
**US-A- 4 749 646**

(73) Proprietor: **EASTMAN KODAK COMPANY (a New Jersey corporation)**
**343 State Street**
**Rochester New York 14650(US)**

(72) Inventor: **Herz, Arthur Herman c/o EASTMAN KODAK COMPANY**
**Patent Department**
**343 State Street**
**Rochester New York 14650(US)**
Inventor: **Burgmaier, George John c/o EASTMAN KODAK COMPANY**
**Patent Department**
**343 State Street**
**Rochester New York 14650(US)**

(74) Representative: **Buff, Michel et al**
**Kodak-Pathé**
**Département des Brevets et Licences CRT**
**Centre de Recherches et de Technologie**
**Zone Industrielle**
**F-71102 Chalon sur Saône Cédex (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

This invention relates to a silver halide photographic emulsion and to a process for preparation thereof. In particular, this invention relates to use of a urea compound as a chemical sensitizing agent for silver halide.

Numerous thiourea compounds have been described as having utility in the photographic art. These uses include sensitization of silver halide. For example, U. S. Patent 1,623,499 (1920) describes thiourea as a useful chemical sensitizing agent for silver halide.

U. S. Patent 4,221,863 discloses the use of substituted thiourea compounds as agents for promoting the growth of silver halide grains during the precipitation and ripening steps. The identified thiourea compounds, typical of which is 1,1,3,3-tetramethyl-2-thiourea, are described as being capable of promoting uniform growth of silver halide grains with respect to both size and crystal habit. However, this patent does not relate to chemical sensitization of silver halide nor does it disclose the type of thiourea compounds which the instant applicants have found to be useful as chemical sensitizing agents for silver halide.

Japanese Public Disclosure 82408/1978 relates to the use of tetrasubstituted thiourea compounds as solvents for silver halide precipitation. The specifically illustrated compounds fail to describe a single tetrasubstituted thiourea compound having the substituents which are necessary to achieve chemical sensitization of silver halide. This is illustrated below by comparative data.

U. S. Patent 3,598,598 describes tetra substituted thiourea compounds as useful emulsion fog-stabilizing agents. Among the described thiourea compounds are those containing carboxysubstituted phenyl groups. This patent contains no disclosure with respect to use of the described compounds as chemical sensitizing agents for silver halide and, as is demonstrated below by comparative data, a carboxyphenyl substituted thiourea compound such as described in the '598 patent is incapable of sensitizing silver halide.

EP 115,351 discloses a silver halide emulsion comprising a combination of a tetrasubstituted thioureas as silver halide solvent and a hardener containing a vinylsulfonyl group. This combination is useful to provide a good storability of the light-sensitive materials. The formulae of the tetrasubstituted thioureas differ from those of the present invention in that the substituents comprising oxygen atoms are alcohols which do not contain any "nucleophilic atom" as defined in the present invention, bonded to a urea nitrogen atom through a 2 or 3 member chain.

Accordingly, the object of the present invention is to provide a photographic silver halide emulsion which comprises a compound which is an effective chemical sensitizing agent for silver halide.

The present invention provides a radiation sensitive silver halide emulsion which comprises a sensitizing amount of a tetrasubstituted urea compound characterized in that said compound has the structural formula:

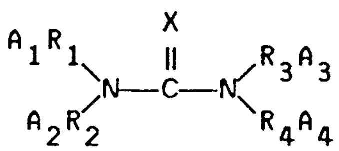

wherein

X is a middle chalcogen atom, i.e., a Group VI A atom below oxygen and above polonium;

each of $R_1$, $R_2$, $R_3$ and $R_4$ independently can represent an alkylene, cycloalkylene, carbocyclic arylene or heterocyclic arylene, alkarylene or aralkylene group; or taken together with the nitrogen atom to which they are attached, $R_1$ and $R_2$ or $R_3$ and $R_4$ complete a 5 to 7 member heterocyclic ring; and

each of $A_1$, $A_2$, $A_3$ and $A_4$ independently is hydrogen or represents a radical comprising a nucleophilic atom that is an oxygen atom of an oxygen acid which is a carboxylic, sulfinic, sulfonic or hydroxamic group, a sulfur atom of a sulfur acid which is a mercaptan, a nitrogen atom of a nitrogen acid which is a sulfonamide, or an unchanged nitrogen atom of an amine

with the proviso that at least one of $A_1R_1$ to $A_4R_4$ contains a nucleophilic atom bonded to a urea nitrogen atom through a 2 or 3 member chain. Sulfur is the preferred Group VIA atom due to ready availability of starting materials for thiourea synthesis and greater solubility of the thiourea compound in aqueous solutions where silver halide sensitization occurs.

Inorganic or organic salts of these acids are equally useful. Oxygen atoms of aliphatic alcohols are excluded from the definition of "nucleophilic atoms" in the present invention.

Preferably, at least one of $R_1A_1$ to $R_4A_4$ is an omega-bound methyl or ethyl carboxylic acid or a salt thereof.

Other than the nucleophilic atom which is necessary for successful chemical sensitization of silver halide and which is attached to the urea nitrogen by two or three atoms, the composition of the remaining RA groups on the 1,1,3,3-tetrasubstituted urea compound can vary widely for achieving the desired chemical sensitization of silver halides. This is shown below by comparative data.

Alkylene groups which can be represented by at least one of $R_1$ to $R_4$ which are not bonded to the required nucleophilic atom can contain from 1 to 6 carbon atoms, preferably from 1 to about 4 carbon atoms for greater solubility properties.

When the $R_1$ to $R_4$ groups are cycloalkylene the ring portion can contain from about 3 to about 8, preferably about 5 or 6 carbon atoms. Where a cycloalkylene group has the required nucleophilic atom bonded thereto it is important for successful operation of this invention that such group be bonded to one of the urea nitrogen atoms through a 2 or 3 member chain.

Where one of the $R_1$ to $R_4$ groups is an aromatic heterocyclic or an aromatic carbocyclic ring, such ring system can comprise from about 5 to about 10 atoms in the ring, such as for example pyrrole, phenyl, naphthyl, pyridinyl, quinolyl and naphthryl. When the aromatic heterocyclic or aromatic carbocyclic group has bonded thereto the required nucleophilic atom, the chain separating the nucleophilic atom from a urea nitrogen atom comprises from 2 to 3 members.

Where an $R_1$ to $R_4$ group is an alkarylene or aralkylene, the alkylene moiety thereof can comprise from about 1 to about 3 carbon atoms and the aryl portion is an aromatic group as described above. When the required nucleophilic atom is bonded to an aralkylene group, the chain separating the nucleophilic atom from a urea nitrogen atom comprises from 2 to 3 atoms.

Heterocyclic rings which can be formed by a urea nitrogen atom with $R_1$ and $R_2$ or with $R_3$ and $R_4$ can comprise 5 or 6 ring members. Typical heterocyclic rings so formed include pyridine, morpholine, piperidine and diazine.

Specific 1,1,3,3-tetrasubstituted-2-thiourea compounds useful in this invention include the following:

1. 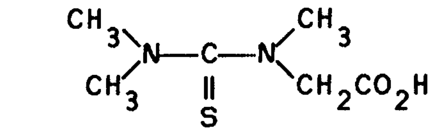

2. 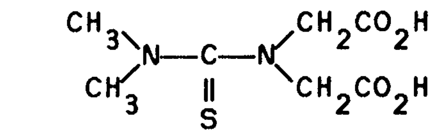

3. 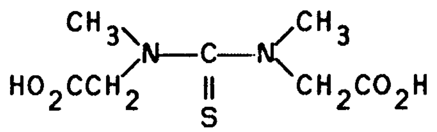

4. 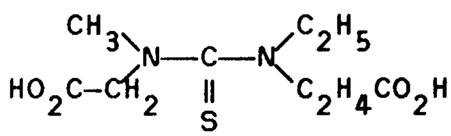

5. 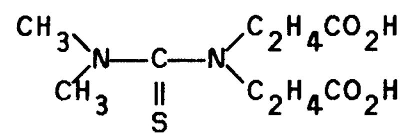

6. 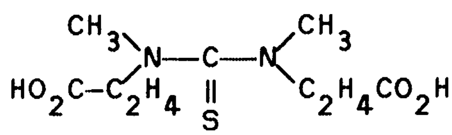

7. 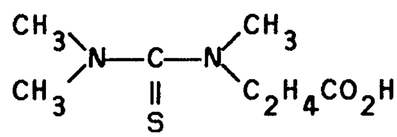

8. 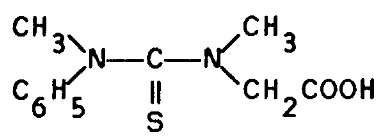

9. 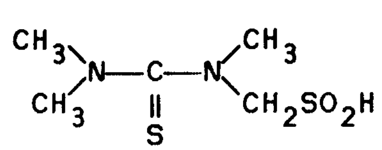

10. 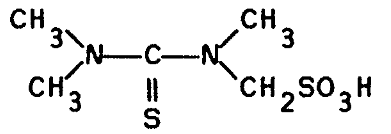

11. 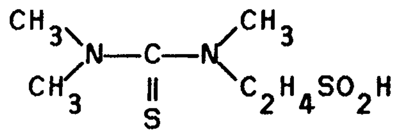

12. 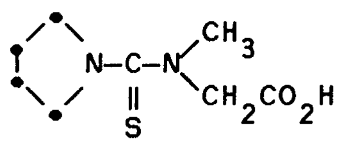

13. 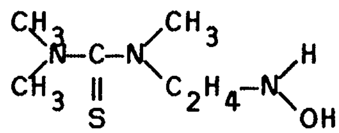

14. 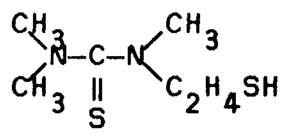

15. 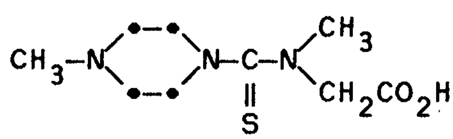

16. 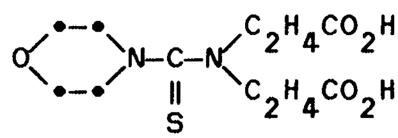

17. 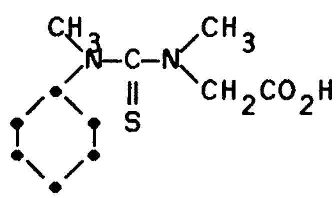

18. 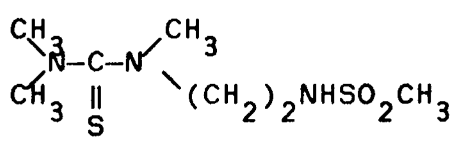

19. 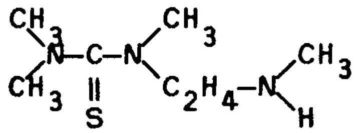

20. 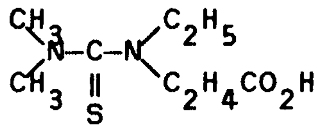

Synthesis of thiourea compounds of this invention can be effected by different techniques known in the art. One method, for example, comprises reacting an aliphatic monoaminocarboxylic acid with a dialkylthiocarbamoyl halide. This method is illustrated below with respect to preparation of Compound I.

Synthesis of Compound 1 (1-carboxymethyl-1,3,3-trimethyl-2-thiourea)

A. To a 500 ml flask was added 24.72 g (0.2 mol) dimethylthiocarbamoyl chloride, 30.72 g (0.2 mol) sarcosine ethyl ester hydrochloride, 300 ml dry acetonitrile and 80 ml (0.44 mol) diisopropylethylamine. The solution was heated with stirring at 55-65 °C for 6.5 hours, cooled to room temperature, and concentrated to dryness. To the residue was added 200 ml ether, 200 ml water and 20 ml 12N HCl. The layers were separated and the aqueous phase extracted twice more with ether. The combined ether solutions were washed with 100 ml of 1 N HCl and then with water. After drying over anhydrous magnesium sulphate, the solvent was removed, followed by distillation of the product, bp 130 °C at 0.15mm Hg. The yield was 27.38g (67%). The NMR, IR, and combustion analyses were consistent with the assigned structure for 1-ethoxycarbonylmethyl-1,3,3- trimethyl-2-thiourea.

B. To a solution of 75 ml water and 150 ml acetone was added 6g (0.15 mol) of sodium hydroxide and 10.22g (0.05 mol) of the above described 1-ethoxycarbonylmethyl-1,3,3-trimethyl-2-thiourea. The solution was stirred at room temperature for 2.5 hours. After cooling in an ice bath, 13 ml of 12N HCl in 15 ml water was added. The solvent was removed at 50 °C under water aspirator pressure. 50 ml of isopropanol alcohol were added to the residue. The suspension was heated to reflux followed by decanting of the clear liquid. This step was repeated twice more with isopropanol alcohol. The isopropanol alcohol solutions were combined and concentrated in vacuo, leaving an oil. The combustion analyses, NMR, and IR were consistent with the assigned structure for Compound 1.

This invention also provide a process for sensitizing a silver halide emulsion which is formed according to processes generally well known in the art. A double jet type process is preferred. The silver halide grains can comprise mixed or single halide components and especially include chloride, bromide, iodide, iodochloride, iodobromide or chlorobromide grains.

The double jet process comprises adding an aqueous silver nitrate solution and an aqueous solution of one or more halides, for example an alkali metal halide such as potassium bromide, potassium chloride, potassium iodide or mixtures thereof, simultaneously to a stirred solution of a silver halide protective colloid through two separate jets.

In the present invention the described sensitizing urea compounds may be added to a silver halide emulsion at various stages during its preparation. For example, the compounds may be added at levels from about $10^{-6}$ to about $10^{-2}$mol thereof per mol of silver halide. A preferred concentration of urea compound to achieve sensitizing of silver halide is from about $10^{-5}$ to about $10^{-3}$mol thereof per mol of silver halide.

The urea sensitizing compounds may be added singly or in combination with other urea compounds, including other sensitizing agents. They may also be added to a silver halide emulsion along with silver ion ligands and silver halide growth modifiers or stabilizers and antifogging agents, or with spectral or chemical sensitizing agents, such as salts or complexes comprising iridium or gold, during formation of silver halide grains, during the physical or chemical ripening stage, or in a separate step before coating.

Conditions for sensitizing silver halide grains such as pH, pAg, temperature, etc., are not particularly limited when employed using compounds described herein. The pH is generally about 1 to 9, preferably about 2 to 6, and pAg is generally about 5 to about 12, preferably from about 7 to about 10. Silver halide grains may be sensitized at temperatures between about 30° to about 90°C., with about 35°C to about 70°C being preferred.

Gelatin is preferred as the binder or protective colloid for the photographic emulsion of the present invention. However, other hydrophilic colloids are also suitable. For example, proteins such as gelatin derivatives, graft polymers of gelating and other polymers, albumin, casein, cellulose derivatives such as hydroxyethyl cellulose, carboxymethyl cellulose, cellulose sulfate, sugar derivatives such as sodium alginate, starch derivatives and various synthetic peptiziers such as hydrophilic homopolymers or copolymers such as polyvinyl alcohol, poly-N-vinylpyrrolidone, polyacrylic acid, polymethyacrylic acid, polyacrylamine, polyvinylimidazole, polyvinyl pyrazole can be used.

Acid-processed gelatin can be used as well as lime-processed gelatin. Futher, gelatin hydrolyzates, and enzyme-hydrolyzed products of gelatin are also usable.

Surface active agents may be incorporated in a photographic emulsion layer or in another hydrophilic colloid layer as a coating aid to prevent build-up of static charge, to improve lubrication properties, to improve emulsion dispersion, to prevent adhesion, and to improve such photographic charcteristics as acceleration of development, increase in contrast, or sensitization.

A photographic emulsion of the present invention may contain antifogging agents or emulsion stabilizing agents, such as for example azaindenes, thionamides, azoles and the like.

The photographic emulsion of the present invention may be spectrally sensitized with dyes. Dyes which can be used include cyanine dyes, merocyanine dyes, composite cyanine dyes, composite merocyanine dyes, and hemioxonol dyes. Particularly useful dyes are those belonging to the merocycanine class. These dyes contain as a basic heterocyclic ring nucleus any nucleus ordinarily used in cyanine dyes.

The photographic emulsion of the present invention may contain color image-forming couplers, i.e., compounds capable of reacting with an oxidation product of an aromatic amine (usually a primary amine) to form a dye. Non-diffusing couplers containing a ballast group are desirable. Either 4-equivalent and 2-equivalent couplers are usable. In addition, colored couplers showing the effect of color correction, or couplers releasing a development inhibitor upon development (so-called DIR couplers) may be used.

A photographic emulsion of the present invention is coated on a support conventionally used for photographic light-sensitive materials such as a flexible support (e.g., plastic film, paper, etc.) or a rigid support (e.g., glass, etc.) according to a dip-coating method, roller coating method, curtain coating method or extrusion coating method.

Emulsions of the present invention can be applied to a multilayer multicolor photographic material comprising a support having provided thereon at least two layers having different spectral sensitivites. Multilayer multicolor photographic materials usually comprise a support having provided thereon at least one red-sensitive emulsion layer, at least one green-sensitive emulsion layer, and at least one blue-sensitive emulsion layer. The order of these layers can optionally be selected as occasion demands. Usually, a cyan-forming coupler is associated with the red-sensitive emulsion layer, a magenta-forming coupler is associated with the green-sensitive emulsion layer, and a yellow-forming coupler is associated with the blue-sensitive emulsion layer. In some cases, however, different layer arrangements may be employed.

The photographic emulsions obtained by the present invention can be processed according to known methods. A developer to be used for the black-and-white processing can contain conventional developing agents such as dihydroxybenzenes (e.g., hydroquinone), 3-pyrazolidones (e.g., 1-phenyl-3-pyrazolidone), aminophenols (e.g., N-Methyl-p-aminophenol), 1-phenyl-3-pyrazolines or ascorbic acids.

As color-developing agent, there can be used primary aromatic amine developing agents such as phenylenediamines (e.g., 4-amino-N,N-diethylaniline, 3-methyl-4-amino-N,N-diethylaniline, 4-amino-N-ethyl-N-hydroxy-ethylaniline, 3-methyl-4-amino-N-ethyl-N-hydroxyethylaniline, 3-methyl-4-amino-N-ethyl-N-methanesulfonamidoethylaniline and 4-amino-3-methyl-N-ethyl-N-methoxyethylaniline. In addition, the developing agents described in L.F.A. Mason, Photographic Processing Chemistry (Focal Press, 1966), pp. 226-229, as well as those described in U.S. Patent Nos. 2,193,015 and 2,592,364 may be used.

A photographic emulsion of the present invention can be applied to many different silver halide photographic light-sensitive materials due to its high photographic sensitivity, contrast, and fog reduction. For example, it can be used in high speed black-and-white negative films, in X-ray films and in multilayer color negative films.

The following examples further illustrate the invention. All parts, percents and ratios are by weight unless otherwise specified.

Example 1

0.80μm octahedral AgBr emulsions (pH 6.3, pBr 3.0), each containing 0.01 mmol/mol Ag of a thiourea compound indicated below, were heated for 30 minutes at 80°C, cooled and coated on a film support at 5813mg Ag/$m^2$ and 10,018mg gel/$m^2$. The coatings were dried and exposed (0.1 sec., 500W 3000°K)

through a graduated density step wedge, processed (5 min. 20 °C) in Eastman Kodak Developer DK-50, washed and dried. The fog in each emulsion was less than 0.08 density unit. The relative speeds of compounds of the invention and of structurally similar compounds known in the art are shown below in Table 1.

TABLE I

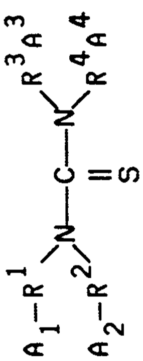

| Thiourea Compound | $A^1$ | $R^1$ | $A^2$ | $R^2$ | $A^3$ | $R^3$ | $A^4$ | $R^4$ | Relative Speed |
|---|---|---|---|---|---|---|---|---|---|
| None (control) | - | - | - | - | - | - | - | - | 100 |
| A (comparison) | - | H | - | H | - | H | - | H | 120 |
| B (comparison) | H | $CH_2$ | H | $CH_2$ | H | $CH_2$ | H | $CH_2$ | 97 |
| C (comparison) | H | $CH_2$ | p-$CO_2H$ | $C_6H_4$ | H | $CH_2$ | p-$CO_2H$ | $C_6H_4$ | 95 |
| D (comparison) | $CO_2H$ | $C_3H_6$ | H | $CH_2$ | H | $CH_2$ | H | $CH_2$ | 102 |
| E (comparison) | $CO_2H$ | $C_3H_6$ | H | $CH_2$ | H | $CH_2$ | $CO_2H$ | $C_3H_6$ | 95 |
| 1 (invention) | $CO_2H$ | $CH_2$ | H | $CH_2$ | H | $CH_2$ | H | $CH_2$ | 316 |
| 3 (invention) | $CO_2H$ | $CH_2$ | H | $CH_2$ | H | $CH_2$ | $CO_2H$ | $CH_2$ | 316 |
| 6 (invention) | $CO_2H$ | $C_2H_4$ | H | $CH_2$ | H | $CH_2$ | $CO_2H$ | $C_2H_4$ | 417 |

From the above results it is apparent that carboxymethyl (Compounds 1 and 3) and carboxyethyl (Compound 6) groups appreciably enhance the chemical sensitivity of otherwise ineffective thiourea and tetramethylthiourea compounds (A and B, respectively). The critical size of the divalent alkylene groups (R) of the compounds useful in this invention in comparison with other similar compounds is apparent by

comparison of results from Compounds 6 and E. The addition of a single methylene ($-CH_2-$) group to the alkylene chains of Compound 6 drastically alters the chemical sensitization properties. Carboxyphenyl substitution on the thiourea structure fails to sensitize silver halide as is shown by Compound C results.

Example 2

The same emulsions and conditions were used as in Example 1 except that instead of the 30 minutes heat treatment at 80 °C, the emulsions were kept for 120 minutes at 40 °C before coating. The compounds employed are identified in Table I.

TABLE II

| Thiourea Compound | Relative Speed |
|---|---|
| None (Control) | 100 |
| A | 138 |
| 6 | 661 |

Table II results make it apparent that compared to conventional chemical sensitizers like thiourea, a substituted thiourea of this invention is more effective for improving emulsion speed under relatively mild conditions.

Example 3

8 mM cubic AgBr was dispersed at pH 3, pBr 3 in 0.02% ossein gelatin containing 28 mM $KNO_3$ and 0.6 mM of a thiourea compound. Following 17 hours agitation at 25 °C, electronmicrographs were obtained and AgBr particle dimensions were evaluated with a Zeiss Particle Size Analyzer, MOP III. Results are reported in Table III. The compounds employed are identified above in Table I.

TABLE III

| Compound | Equivalent Circular Diameter, in microns | Standard Deviation, microns |
|---|---|---|
| None (Control) | 0.166 | 0.004 |
| B | 0.203 | 0.005 |
| 3. | 0.164 | 0.003 |
| 6. | 0.168 | 0.004 |

The results show that whereas tetramethyl thiourea (Compound B), a known Ostwald ripener, increases AgBr particle size, the thiourea compounds disclosed herein had no detectable influence on crystal dimensions.

Example 4

An octahderal AgBr emulsion containing on average 0.5 micrometer crystals and with 40 g ossein gelatin/mol Ag, was given a sensitization treatment as noted below for 40 minutes at 70 °C. The emulsions were coated at 2153 mg Ag and 3229 mg gelatin per $m^2$. The dried coatings were then exposed sensitometrically at 365 nm for 0.1 second and processed for 6 minutes in KODAK Rapid X-Ray Developer. Fog densities did not exceed 0.06. The tabulated speed values were normalized with respect to the control.

| Thiourea Sensitizing Agent (mg/Ag mol) | Relative Speed |
|---|---|
| Control (None) | 100 |
| Compound 3 (2 mg) | 372 |
| Compound 3 (2 mg), $KAuCl_4$ (4 mg) | 1510 |

These results make it apparent not only that a thiourea compound as described herein is by itself an effective sensitizer, but that it provides enhanced speed in combination with an ionic gold, chemical sensitizer.

Example 5

Silver halide emulsions containing cubic crystals of 0.29 micrometer edge length were given a sensitization treatment for 0.5 hours at 55 °C. After cooling to 40 °C the emulsions were coated at 3229 mg Ag and 7535 mg gelation per $m^2$. The dried coatings were exposed sensitometrically for 0.1 second and processed for 12 minutes in KODAK developer DK50. Fog densities did not exceed 0.1. The determined relative speeds shown below were normalized with respect to the control which contained no added sensitizing agent.

| Thiourea Sensitizer 0.46 mg/mol Ag | Relative Speed |
|---|---|
| Control (No sensitizer) | 100 |
| Compound 3 | 562 |

It is clear from these results that a thiourea of this invention can act as a highly effective AgCl sensitizer.

**Claims**

**1.** A photographic silver halide emulsion comprising a sensitizing amount of a tetra-substituted urea compound characterized in that said compound has the structural formula :

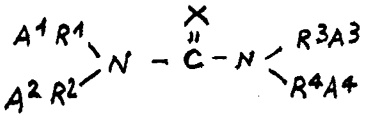

wherein

X is a middle chalcogen atom, i.e., a Group VI A atom below oxygen and above polonium ;

each of $R_1$, $R_2$, $R_3$ and $R_4$ independently can represent an alkylene, cycloalkylene, carbocyclic arylene or heterocyclic arylene, or aralky group ; or taken together with the nitrogen atom to which they are attached, $R_1$ and $R_2$ or $R_3$ and $R_4$ complete a 5 to 7 member heterocyclic ring ; and

each of $A_1$, $A_2$, $A_3$ and $A_4$ independently is hydrogen or represents a radical comprising a nucleophilic atom that is,

AN OXYGEN ATOM OF AN OXYGEN ACID WHICH IS A CARBOXYLIC, SULFINIC, SULFONIC, OR HYDROXAMIC GROUP,

A SULFUR ATOM OF A SULFUR ACID WHICH IS A MERCAPTAN,

A NITROGEN ATOM OF A NITROGEN ACID WHICH IS A SULFONAMIDE,

OR AN UNCHARGED NITROGEN ATOM OF AN AMINE,

with the proviso that at least one of $A_1R_1$ to $A_4R_4$ contains a nucleophilic atom bonded to a urea nitrogen atom through a 2 or 3 member chain.

2. The photographic emulsion of claim 1, characterized in that X is sulfur.

3. The photographic emulsion of claim 1 or 2, characterized in that said nucleophilic atom is the oxygen of a carboxylic acid, or of an inorganic or organic salt thereof.

4. The photographic emulsion of claim 1 - 3, characterized in that said thiourea compound is present in an amount of from $10^{-6}$ to $10^{-2}$ mol thereof per mole of silver halide.

5. The photographic emulsion of claim 4, characterized in that the thiourea compound is present in an amount of from $10^{-5}$ to $10^{-3}$ mole thereof per mol of silver halide.

6. The photographic emulsion of any of claims 1 - 5, characterized in that the thiourea compound is :

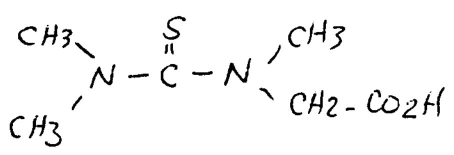

7. The photographic emulsion of any of claims 1 - 5, characterized in that the thiourea compound is :

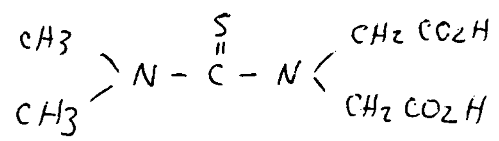

8. The photographic emulsion of any of claims 1 - 5, characterized in that the thiourea compound is :

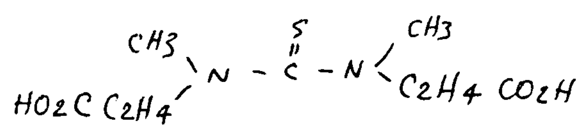

## Patentansprüche

1. Photographische Silberhalogenidemulsion mit einer sensibilisierenden Menge einer tetra-substituierten Harnstoff-Verbindung, dadurch gekennzeichnet, daß diese Verbindung der folgenden Strukturformel entspricht:

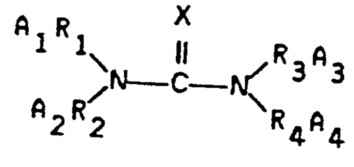

worin bedeuten:
X ein mittleres Chalcogenatom, d. h., ein Atom der Gruppe VI A unterhalb Sauerstoff und oberhalb Polonium;
$R_1$, $R_2$, $R_3$ und $R_4$ jeweils unabhängig voneinander eine Alkylen-, Cycloalkylen-, carbocyclische Arylen- oder heterocyclische Arylen- oder Aralkylgruppe; oder gemeinsam mit dem Stickstoffatom, an dem sie sitzen, können $R_1$ und $R_2$ oder $R_3$ und $R_4$ einen 5- bis 7-gliedrigen heterocyclischen Ring vervollstän-

digen; und
$A_1$, $A_2$, $A_3$ und $A_4$ jeweils unabhängig voneinander Wasserstoff oder einen Rest mit einem nukleophilen Atom, d. h.,
einem Sauerstoffatom einer Sauerstoffsäure, die eine carbocyclische, sulfinische, sulfonische oder Hydroxamingruppe darstellt,
einem Schwefelatom einer Schwefelsäure, bei der es sich um ein Mercaptan handelt,
einem Stickstoffatom einer Stickstoffsäure, wobei es sich um ein Sulfonamid handelt,
oder einem ungeladenen Stickstoffatom eines Amins,
wobei gilt, daß mindestens einer der Reste $A_1R_1$ bis $A_4R_4$ ein nukleophiles Atom aufweist, das an ein Harnstoff-Stickstoffatom durch eine 2- oder 3-gliedrige Kette gebunden ist.

**2.** Photographische Emulsion nach Anspruch 1, dadurch gekennzeichnet, daß X für Schwefel steht.

**3.** Photographische Emulsion nach Anspruch 1 oder 2, dadurch gekennzeichnet, daß das nukleophile Atom das Sauerstoffatom einer Carboxylsäure ist oder eines anorganischen oder organischen Salzes hiervon.

**4.** Photographische Emulsion nach Anspruch 1 - 3, dadurch gekennzeichnet, daß die Thioharnstoff-Verbindung in einer Menge von $10^{-6}$ - $10^{-2}$ Mol pro Mol Silberhalogenid vorliegt.

**5.** Photographische Emulsion nach Anspruch 4, dadurch gekennzeichnet, daß die Thioharnstoff-Verbindung in einer Menge von $10^{-5}$ - $10^{-3}$ Mol pro Mol Silberhalogenid vorliegt.

**6.** Photographische Emulsion nach einem der Ansprüche 1 - 5, dadurch gekennzeichnet, daß die Thioharnstoff-Verbindung der folgenden Formel entspricht:

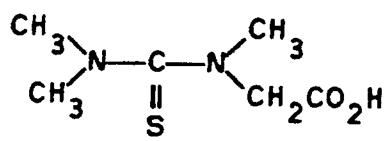

**7.** Photographische Emulsion nach einem der Ansprüche 1 - 5, dadurch gekennzeichnet, daß die Thioharnstoff-Verbindung der folgenden Formel entspricht:

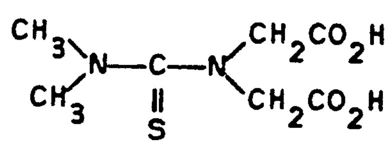

**8.** Photographische Emulsion nach einem der Ansprüche 1 - 5, dadurch gekennzeichnet, daß die Thioharnstoff-Verbindung der folgenden Formel entspricht:

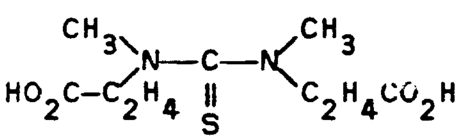

## Revendications

**1.** Emulsion photographique aux halogénures d'argent comprenant une quantité appropriée pour la sensibilisation d'un composé thiourée tétra-substitué caractérisée en ce que le composé a la formule :

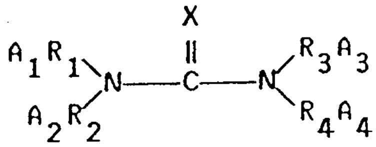

où

X est un atome chalcogène moyen, c'est à dire un atome du groupe VIA situé en-dessous de l'oxygène et au-dessus du polonium ;

chaque groupe $R_1$, $R_2$, $R_3$ et $R_4$ séparément représente un alkylène, cycloalkylène, arylène carbocyclique ou arylène hétérocyclique, ou un groupe aralky ; ou ensemble avec l'atome d'azote auxquels ils sont rattachés, $R_1$ et $R_2$ ou $R_3$ et $R_4$ complètent un hétérocycle à 7 chaînons ; et

chaque $A_1$, $A_2$, $A_3$ et $A_4$ séparément est un hydrogène ou représente un radical comprenant un atome nucléophile qui est,

un atome d'oxygène d'un acide oxygéné qui est un groupe carboxylique, sulfinique, sulfonique ou hydroxamique,

un atome de soufre d'un acide soufré qui est un mercaptan,

un atome d'azote d'un acide azoté qui est un sulfonamide,

ou un atome d'azote ne portant pas de charge d'une amine,

avec la condition qu'au moins un des groupes $A_1R_1$ à $A_4R_4$ contienne un atome nucléophile lié à l'atome d'azote de l'urée au moyen d'une chaîne à 2 ou 3 membres.

2. Emulsion photographique selon la revendication 1, caractérisée en ce que X est le soufre.

3. Emulsion photographique selon la revendication 1 ou 2, caractérisée en ce que l'atome nucléophile est l'atome d'oxygène d'un acide carboxylique ou d'un sel organique ou inorganique de celui ci.

4. Emulsion photographique selon les revendications 1 à 3, caractérisée en ce que le composé thiourée est présent en quantité comprise entre $10^{-6}$ et $10^{-2}$ mole par mole d'halogénure d'argent.

5. Emulsion photographique selon la revendication 4, caractérisée en ce que le composé thiourée est présent en quantité comprise entre $10^{-5}$ et $10^{-3}$ mole par mole d'halogénure d'argent.

6. Emulsion photographique selon l'une quelconque des revendications 1 à 5, caractérisée en ce que le composé thiourée est :

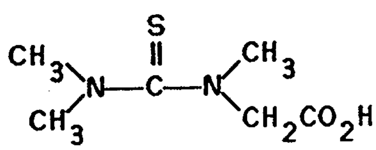

7. Emulsion photographique selon l'une quelconque des revendications 1 à 5, caractérisée en ce que le composé thiourée est :

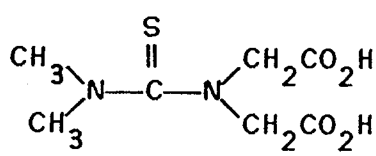

8. Emulsion photographique selon l'une quelconque des revendications 1 à 5, caractérisée en ce que le composé thiourée est :